# FASCICULE DE BREVET EUROPEEN

(11) **EP 1 511 728 B1**
(45) Date de publication et mention de la délivrance du brevet: **14.03.2007**
(21) Numéro de dépôt: 03755201.5
(22) Date de dépôt: 26.05.2003
(51) Int. Cl.: C07D 211/62, C07D 207/16, C07D 211/16, C07D 295/18, C07D 211/22, C07D 209/44, C07D 211/20, C07D 211/52, C07D 211/64, C07D 217/02, C07D 471/10, A61K 31/4545, A61P 29/00

(54) **DERIVES D OXOPHENYL-CYCLOHEXYL-PROPANOLAMINE, LEUR PREPARATI ON ET LEUR APPLICATION EN THERAPEUTIQUE**
OXOPHENYLCYCLOHEXYLPROPANOLAMINDERIVATE, DEREN HERSTELLUNG UND VERWENDUNG IN THERAPEUTIKA
OXOPHENYL-CYCLOHEXYL-PROPANOLAMINE DERIVATIVES, PRODUCTION AND USE THEREOF IN THERAPEUTICS

(30) Priorité: 29.05.2002 FR 0206560
(43) Date de publication de la demande: 09.03.2005
(73) Titulaire: Sanofi-Aventis, 75013 Paris (FR)
(72) Inventeur: BOVY, Philippe, R., F-78750 Mareil Marly (FR); CECCHI, Roberto, I-26900 Lodi (IT); CROCI, Tiziano, I-20155 Milan (IT); VENIER, Olivier, F-92500 Rueil Malmaison (FR)
(74) Mandataire: Romanowski, Caroline
(86) Numéro de dépôt international: PCT/FR2003/001579
(87) Numéro de publication internationale: WO 2003/099819

(56) Documents cités:
- WO-A-01/07026
- WO-A-01/44187
- WO-A-02/44139

## Description

La présente invention se rapporte à des dérivés d'oxophényl-cyclohexylpropanolamine, à leur préparation et à leur application en thérapeutique.

WO01/07026 décrit des composés de formule : 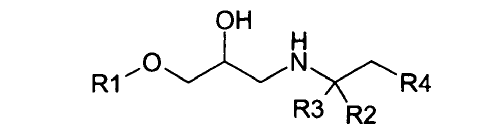 dans laquelle R1 peut être un phényle éventuellement substitué, R2 et R3 représentent un atome d'hydrogène ou un C1-C4alkyle, et R4 peut représenter un phényle éventuellement substitué, en tant qu'agonistes des récepteurs β3.

La présente invention a pour objet des composés répondant à la formule (I) : 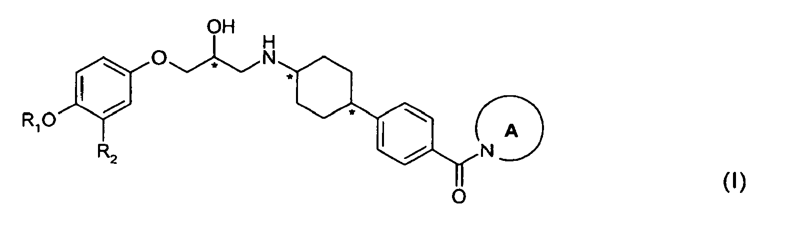 dans laquelle :
**R₁** représente un atome d'hydrogène ou un groupe (C1-C4)alkyle, un groupe -CO(C1-C4)alkyle, un groupe (C1-C4)alkyl-phényle ou un groupe -CO-phényle, ledit phényle étant éventuellement substitué par un à trois groupes choisis indépendamment les uns des autres parmi les atomes d'halogène, les groupes (C1-C4)alkyles et (C1-C4)alcoxy ;
**R₂** est choisi parmi l'un des groupes suivants :
   - un atome d'hydrogène,
   - un atome d'halogène,
   - un groupe -S(O)_{z}R₃,
   - un groupe -NHSO₂R₃,
   - un groupe -NHSO₂-phényle, ou
   - un groupe -NHSO₂-(C1-C4)alkyl-phényle,
où z est égal à 0, 1 ou 2 et où R₃ représente un groupe (C1-C4)alkyle, ledit phényle étant éventuellement substitué par un à trois groupes choisis indépendamment les uns des autres parmi les atomes d'halogène, les groupes (C1-C4)alkyles et (C1-C4)alcoxy; et
**A** est choisi parmi l'un des groupes de formule : 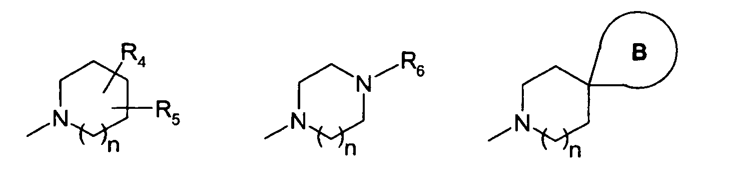 dans lesquelles :
- n est égal à 0, 1 ou 2,
- R₄ et R₅ sont portés soit par des atomes de carbone différents, soit par le même atome de carbone du cycle auquel ils sont rattachés, et sont choisis indépendamment l'un de l'autre parmi les groupes suivants : un atome d'hydrogène, un groupe (C1-C4)alkyle, hydroxy, cyano, phényle, benzyle, pipéridyle, -CONH₂, -CO-phényle, -COOR₃ (où R₃ est tel que défini ci-dessus), -CH(phényl)(OH) et -C(phényl)₂(OH), au moins l'un de R₄ ou R₅ étant différent d'un atome d'hydrogène,
- soit R₄ et R₅ sont portés par des atomes de carbone adjacents du cycle auquel ils sont rattachés et forment ensemble, avec les atomes de carbone qui les portent, un cycle aromatique à 6 chaînons éventuellement substitué par 1 à 3 groupes (C1-C4)alkyles ou (C1-C4)alcoxy,
- R₆ représente un atome d'hydrogène ou un groupe (C1-C4)alkyle, phényle ou benzyle, et
- B représente un groupe cycloalkyle à 5 ou 6 chaînons, saturé ou insaturé, comportant éventuellement 1 ou 2 atomes d'azote, ce groupe cycloalkyle étant lui-même fusionné avec un groupe phényle ou substitué par un à trois groupes choisis parmi les groupes phényle et carbonyle.

Les composés de formule (I) peuvent comporter un ou plusieurs atomes de carbone asymétriques. Par ailleurs, le groupe cyclohexyle de ces composés présente une asymétrie géométrique. Les signes * dans la formule (I) ci-dessus désignent les carbones qui peuvent donner lieu à différentes configurations géométriques.
Les composés de formule (I) peuvent donc exister sous forme d'énantiomères ou de diastéréoisomères. Ces énantiomères, diastéréoisomères, ainsi que leurs mélanges, y compris les mélanges racémiques, font partie de l'invention.
On préfère notamment les composés de formule (I) selon l'invention qui présentent la configuration suivante :

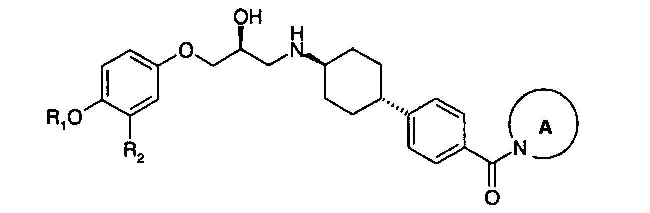

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe (C1-C4)alkyle : un groupe aliphatique saturé linéaire ou ramifié comprenant de 1 à 4 atomes de carbone (étant bien entendu qu'un tel groupe ne peut être que linéaire lorsqu'il comprend moins de 3 atomes de carbone, et qu'un tel groupe peut être linéaire ou ramifié lorsqu'il comprend 3 ou 4 atomes de carbone). A titre d'exemples, on peut citer les groupes méthyle, éthyle, propyle, isopropyle, butyle, isobutyle, etc ;
- un groupe (C1-C4)alcoxy : un radical -O-(C1-C4)alkyle, où le groupe (C1-C4)alkyle est tel que précédemment défini ; et
- un groupe (C1-C4)alkyl-phényle : un groupe de formule -(CH₂)ₓ-phényle, où x est compris entre 1 et 4.

Parmi les composé de formule (I) objets de l'invention, on peut citer les composés préférés dans lesquels :
R₁ représente un atome d'hydrogène;
et/ou R₂ représente un groupe -SO₂R₃ ou -NHSO₂R₃, où R₃ est tel que précédemment défini (R₃ représentant avantageusement un groupe méthyle ou un groupe butyle) ;
et/ou A est choisi parmi l'un des groupes de formule : 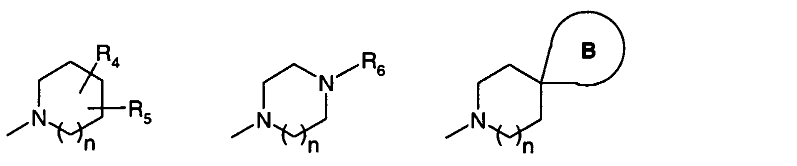
dans lesquelles n est égal à 0 ou 1, R₄, R₅, R₆ et B étant tels que définis ci-dessus.

Parmi les composé de formule (I) objets de l'invention, d'autres composés préférés sont ceux dans lesquels :
R₁ représente un atome d'hydrogène ;
R₂ représente un groupe -SO₂R₃ ou -NHSO₂R₃, où R₃ est tel que précédemment défini (R₃ représentant avantageusement un groupe méthyle) ; et
A est choisi parmi l'un des groupes suivants :
   - un groupe de formule 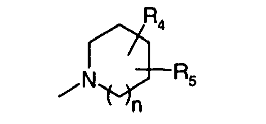
dans laquelle n est égal à 0 ou 1 et R₄ et R₅ sont portés soit par des atomes de carbone différents, soit par le même atome de carbone du cycle auquel ils sont rattachés, et sont choisis indépendamment l'un de l'autre parmi les groupes suivants : un atome d'hydrogène, un groupe (C1-C4)alkyle, hydroxy, cyano, phényle, benzyle, pipéridyle, -CONH₂, -CO-phényle, -COOR₃ (où R₃ est tel que défini ci-dessus), - CH(phényl)(OH) et -C(phényl)₂(OH), au moins l'un de R₄ ou R₅ étant différent d'un atome d'hydrogène,
- un groupe de formule 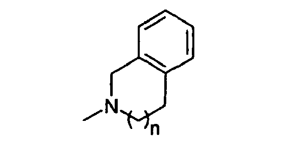
dans laquelle n est égal à 0 ou 1 et le cycle aromatique est éventuellement substitué par 1 à 3 groupes choisis indépendamment les uns des autres parmi les groupes (C1-C4)alkyles et (C1-C4)alcoxy,
- un groupe de formule 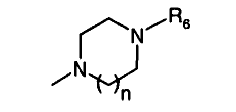
dans laquelle n est égal à 0 ou 1 et R₆ représente un atome d'hydrogène ou un groupe benzyle, et
- un groupe de formule 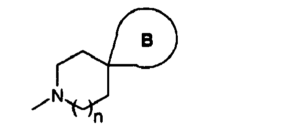
dans laquelle n est égal à 0 ou 1 et B représente un groupe cycloalkyle à 5 ou 6 chaînons, saturé ou insaturé, comportant éventuellement 1 ou 2 atomes d'azote, ce groupe cycloalkyle étant lui-même fusionné avec un groupe phényle ou substitué par un à trois groupes choisis parmi les groupes phényle et carbonyle.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective groups in Organic Synthesis », Green *et al.,* 2^{nd} Edition (John Wiley & Sons, Inc., New York).

Conformément à l'invention, on peut préparer les composés de formule générale (I) selon le procédé décrit dans le schéma 1.

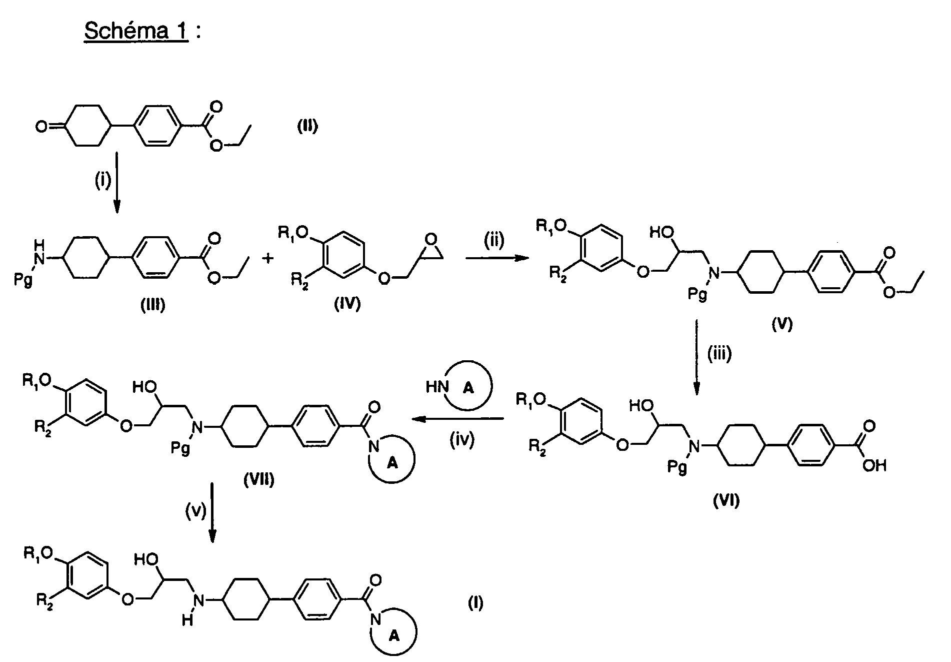

Selon le schéma 1, on transforme la fonction cétone du composé de formule (II), dans une étape (i), en un groupe amine selon les méthodes bien connues de l'Homme du métier (par exemple par amination réductrice). L'amine (III) se trouve partiellement protégée à l'aide d'un groupe protecteur Pg, tel qu'un groupe benzyle éventuellement substitué (par exemple un groupe para-méthoxybenzyle) ou un groupe méthoxyéthoxyméthyle (MEM). On préfère ici utiliser des groupes protecteurs qui ne protègent que partiellement la réactivité de la fonction amine, c'est-à-dire qui n'altèrent pas son caractère nucléophile. Cependant, dans le cas où R₂ représente un groupe SOR₃ où R₃ est un groupe (C1-C4)alkyle, on utilisera de préférence, en tant que produit de départ, un composé (III) portant une amine primaire (Pg = H dans le schéma 1).
Dans une étape (ii), on fait réagir le composé de formule (III) obtenu à l'issue de l'étape (i) avec l'époxyde de formule (IV), dans lequel R₁ et R₂ sont tels que précédemment définis. Les composés de formule (IV) sont connus dans la littérature (par exemple dans la demande de brevet publiée sous le numéro WO 02/44139) ou bien peuvent être préparés par des procédés analogues aux procédés qui y sont décrits. Dans le cas où R₂ peut réagir au cours de cette étape (ii) ou des étapes ultérieures, on le protège préalablement au moyen des groupes protecteurs bien connus de l'Homme du métier.
Lors de l'étape (ii), dans le cas où l'amine primaire est partiellement protégée dans le composé (III), le composé (III) ne peut réagir qu'avec une seule molécule de l'époxyde (IV), et non avec deux molécules, évitant ainsi la formation de sous-produits réactionnels.
L'étape (ii) conduit à l'amino-alcool de formule (V). Cette étape est par exemple réalisée dans un solvant organique, tel qu'un alcool inférieur comme le méthanol, l'éthanol, l'isopropanol ou le tert-butanol, ou encore dans le diméthylsulfoxyde, dans un éther linéaire ou cyclique, dans un amide comme le diméthylformamide ou le diméthylacétamide, ou encore dans un mélange de ces solvants, en utilisant de préférence des quantités au moins équimolaires des réactifs. La température de la réaction est avantageusement comprise entre la température ambiante et la température de reflux du solvant choisi.
Lorsque R₁ représente un atome d'hydrogène, il est préférable de protéger le groupe fonctionnel hydroxy par un groupe protecteur afin d'augmenter le rendement de la réaction. A cet effet, on peut utiliser les groupes protecteurs usuels pour les groupes phénols, tels que le méthoxyéthoxyméthyle (MEM), le triméthylsilyl-éthoxyméthyle (SEM), le benzyle éventuellement substitué, ou le benzoyle.
Dans le cas où R₂ représente un groupe SOR₃ où R₃ est un groupe (C1-C4)alkyle, on protègera de plus l'azote du composé (V) avec un groupement amino-protecteur, comme le t-butyloxycarbonyl (BOC) (Pg = BOC dans le schéma 1 pour les composés (V), (VI) et (VII)), selon les méthodes connues de l'Homme du métier.
Dans une étape (iii), l'ester éthylique du composé (V) obtenu à l'issue de l'étape (ii) est hydrolysé en acide (VI) par traitement par une base, par exemple la soude dans un solvant ou un mélange de solvants, tel qu'un mélange éthanol/eau.
L'amide de formule (VII) est obtenu, dans une étape (iv), en faisant réagir l'acide (VI) avec une amine de formule: 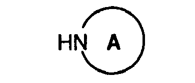 dans laquelle A est tel que défini en rapport avec la formule (I) précédemment décrite, en présence d'un agent de couplage, par exemple le 1-éthyl-3-(3-diméthylaminopropyl)carbodiimide (EDC), l'hexafluorophosphate de benzotriazol-1-yloxytris(diméthylamino)phosphonium (BOP) ou le tétrafluoroborate de benzotriazol-1-yloxy-N, N'-tétraméthyluronium (TBTU), et en présence d'un base telle que la triéthylamine ou la pyridine, dans un solvant tel que le dichlorométhane, l'acétonitrile ou le chloroforme. Il est également possible d'activer la fonction acide du composé (VI) sous forme d'un chlorure d'acide ou d'un anhydride carbonique, selon les techniques connues de l'Homme du métier.
Les composés de formule (I) sont finalement obtenus, dans une étape (v), après élimination des groupes protecteurs au moyen des techniques connues de l'Homme du métier. En particulier, lorsque les groupes protecteurs sont des groupes benzyles, on réalise la déprotection au moyen d'hydrogène en présence de palladium sur charbon, dans un solvant tel que l'éthanol. Dans le schéma 1, il est bien entendu que l'on peut utiliser, en tant que produit (II) de départ, un ester autre qu'un ester éthylique, par exemple un ester méthylique ou propylique ou tout autre ester d'un alkyle inférieur.

La préparation des composés de formule (II), utiles pour la mise en oeuvre du procédé présenté dans le schéma 1 ci-avant. peut être réalisée selon le procédé présenté dans le schéma 2, illustré à titre d'exemple pour la préparation d'un ester éthylique en tant que composé (II).

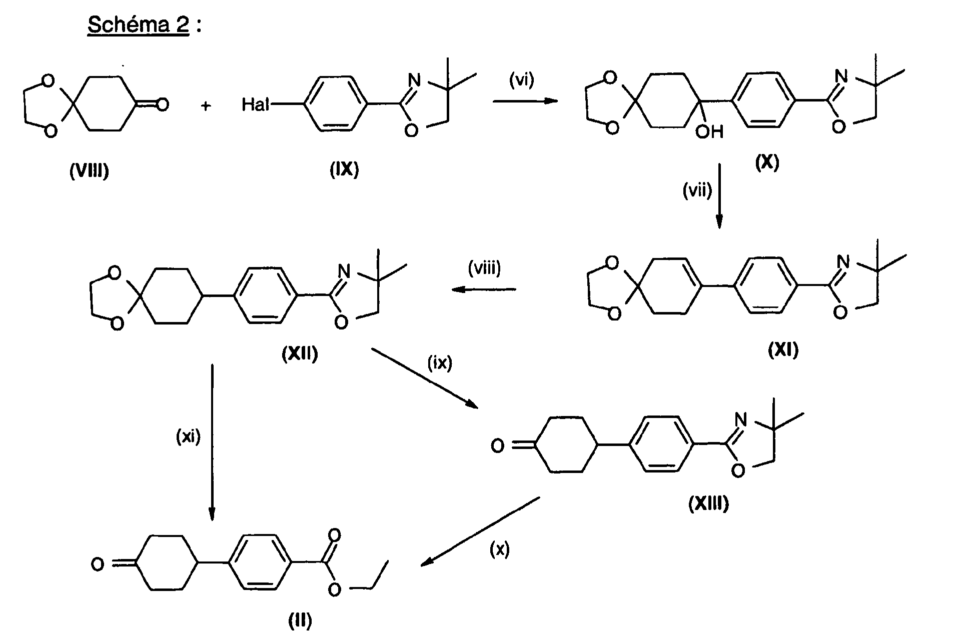

Selon le schéma 2, on procède, dans une étape (vi), à la condensation du composé de formule (VIII) avec le composé de formule (IX), dans lequel Hal représente un atome d'halogène, de préférence le brome, par exemple selon la méthode décrite par MEYERS *et al.* dans J. Org. Chem., 1974, 39, 2787. L'alcool intermédiaire de formule (X) ainsi obtenu est transformé, dans une étape (vii), en composé (XI) insaturé, par exemple à l'aide de SOCl₂ dans la pyridine selon la méthode décrite par GONZALES-CAMENO *et al.* dans Tetrahedron, 1994, 50, 10971 ou bien à l'aide de POCl₃, comme décrit par exemple dans Org. Prep. Proced. Int., 1995, 27, 122.
Le composé (XI) insaturé est ensuite réduit, dans une étape (viii), en composé (XII) selon les méthodes conventionnelles, par exemple à l'aide d'hydrogène en présence de palladium sur charbon, dans un solvant tel que l'éthanol.
L'hydrolyse du groupe acétal du composé (XII) est réalisée, dans une étape (ix), de façon analogue à la réaction décrite par SZANTAY *et al.* dans Tetrahedron, 1996, 52(33), 11053, à savoir à l'aide d'acide chlorhydrique dans l'acétone, et conduit au composé (XIII), qui est ensuite hydrolysé en composé (II), dans une étape (x), selon la méthode décrite par SEEBACH *et al.* dans Synthesis Communications, 1982, 138 ou par NELSON *et al.* dans J. Org. Chem., 1994, 59(9), 2577. Alternativement, le composé (XII) peut être directement converti en composé (II) par chauffage au reflux dans l'éthanol et par addition d'acide sulfurique, selon la méthode décrite par DEGRAW *et al.* dans J. Med. Chem., 1992, 35(2), 320 ou par TAYLOR *et al.* dans Heterocycles, 1996, 43(2), 323.
Il est bien entendu qu'un procédé identique à celui présenté dans le schéma 2 pourrait être mis en oeuvre pour la préparation de composés (II) sous forme d'esters autres que l'ester éthylique, en hydrolysant le composé (XII) à l'aide d'alcools portant des groupes alkyles différents d'un groupe éthyle.

Dans les schémas 1 et 2, les composés de départ et les réactifs, quand leur mode de préparation n'est pas expressément décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Préparation 1: Acide trans 4-[4-(benzyl{(2S)-3-[4-(benzyloxy)-3-[(méthylsufonyl)amino]phénoxy]-2-hydroxypropyl}amino)cyclohexyl]benzoïque

(Produit intermédiaire de formule (VI) où R₁ = Pg = benzyle et R₂ = -NH-SO₂-CH₃)

### 1.1 : Trans 4-[4-(benzylamino)cyclohexyl]benzoate d'éthyle

Une solution de 8.51 ml de benzylamine (77.95 mmoles) et de 16 g de 4-(cyclohexanone)benzoate d'éthyle (64,96 mmoles) dans le triméthylorthoformate (192 ml) est chauffée pendant 18 h à 50°C. Les solvants sont évaporés sous pression réduite et on ajoute 267 ml d'éthanol, puis 2.457 g de borohydrure de sodium. Le mélange réactionnel est laissé sous agitation pendant 2 h. Les solvants sont évaporés sous pression réduite et on ajoute du dichlorométhane et de l'eau. La phase aqueuse est extraite trois fois au dichlorométhane. Les phases organiques sont séchées sur sulfate de magnésium et concentrées sous pression réduite. Le trans 4-[4-(benzylamino)cyclohexyl]benzoate d'éthyle est obtenu sous forme d'une huile (14.69 g, 67%) après purification sur gel de silice (éluant : acétate d'éthyle/éthanol 90/10). [M+H⁺] = 282.2

### 1.2 : Trans 4-[4-(benzyl{(2S)-3-[4-(benzyloxy)-3-[(méthylsulfonyl)amino] phénoxy]-2-hydroxypropyl}amino)cyclohexyl]benzoate d'éthyle

On chauffe au reflux un mélange de 818 mg (1,82 mmoles) de 4-benzyloxy-3-(N-tert-butoxycarbonyl-N-méthylsulfonyl-amino)-1-((2S)-2,3-époxypropoxy)-benzène et 450 mg (1,82 mmoles) de trans 4-[4-(benzylamino)cyclohexyl]benzoate d'éthyle sous forme de base dans 15 ml d'éthanol absolu pendant 16 heures. On refroidit le mélange, on y ajoute 3 ml d'une solution d'éthanol saturée en acide chlorhydrique et on chauffe à 50°C pendant 6 heures. On évapore le solvant et on reprend avec un mélange de 50 ml d'une solution saturée en bicarbonate de sodium et 50 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange chlorure de méthylène/méthanol/NH₄0H (95/5/0,5). On obtient le composé du titre sous forme de solide blanc. [M+H⁺] = 687.

### 1.3 : Acide trans 4-[4-(benzyl{(2S)-3-[4-(benzyloxy)-3-[(méthylsulfonyl) amino]phénoxy]-2-hydroxypropyl}amino)cyclohexyl]benzoïque

On chauffe à 50°C pendant une nuit un mélange de 4.48 g (5.71 mmoles) de 4-{4-[benzyl((2S)-3-{4-(benzyloxy)-3-[(méthylsulfonyl)amino]phénoxy}-2-hydroxypropyl)amino] cyclohexyl}benzoate d'éthyle et de 38 ml d'une solution aqueuse de soude 1 N dans 114 ml d'éthanol. On évapore les solvants, on reprend dans de l'eau et on ajoute doucement une solution d'acide chlorhydrique 1 N jusqu'à pH=1. On filtre et sèche sous vide. On obtient ainsi le composé du titre (4.05 g, 94%) sous forme d'un solide blanc (point de fusion = 160°C).

### Préparation 2 : Acide trans 4-[4-(benzyl{(2S)-3-[4-(benzyloxy)-3-(méthylsulfonyl)phénoxy]-2-hydroxypropyl}amino)cyclohexyl]benzoïque (Produit intermédiaire de formule (VI) où R₁ = Pg = benzyle et R₂ = -SO₂-CH₃)

### 2.1 : Trans 4-[4-(benzyl{(2S)-3-[4-(benzyloxy)-3-(méthylsulfonyl)phénoxy]-2-hydroxypropyl}amino)cyclohexyl]benzoate d'éthyle

On obtient ce produit en procédant comme décrit dans la Préparation 1.2 ci-dessus, en utilisant le trans 4-[4-(benzylamino)cyclohexyl]benzoate d'éthyle et le 4-benzyloxy-3-méthylsulfonyl-1-((2S)-2,3-époxypropoxy)-benzène, décrit dans la demande de brevet WO 99/65895, et sans y ajouter la solution d'acide chlorhydrique dans l'éthanol. [M+H⁺] = 672.

### 2.2: Acide trans 4-[4-(benzyl{(2S)-3-[4-(benzyloxy)-3-(méthylsulfonyl) phénoxy]-2-hydroxypropyl}amino)cyclohexyl]benzoïque

On procède de façon similaire à la Préparation 1.3 ci-dessus, mais en utilisant le trans 4-[4-(benzyl{(2S)-3-[4-(benzyloxy)-3-(méthylsulfonyl)phénoxy]-2-hydroxypropyl} amino)cyclohexyl]benzoate d'éthyle. On obtient ainsi 8.13 g (93%) du composé du titre sous forme d'un solide blanc (point de fusion = 128-130°C).

### Exemple 1: Trans N-[5-({(2S)-3-[(4-{4-[(4-benzylpipéridin-1-yl)carbonyl] phényl}cyclohexyl)amino]-2-hydroxypropyl}oxy)-2-hydroxyphényl] méthane sulfonamide (composé n° 4)

### 1.1 : N-[5-({(2S)-3-[benzyl(4-{4-[(4-benzylpipéridin-1-yl)carbonyl]phényl} cyclohexyl)amino]-2-hydroxypropyl}oxy)-2-(benzyloxy)phényl] méthane sulfonamide

Une solution de 2 g (2.88 mmol) d'acide trans 4-{4-[benzyl((2S)-3-{4-(benzyloxy)-3-[(méthylsulfonyl)amino]phénoxy}-2-hydroxypropyl)amino]cyclohexyl}benzoïque (Préparation 1), de 0.78 g (5.76 mmol) de 1-hydroxy-benzotriazole, de 1.1 g (5.76 mmol) de 1-éthyl-3-(3-diméthylamino-propyl) carbodiimide, de 1.2 ml de triéthylamine et de 1.01 ml (5.76 mmol) de 4-benzyl-pipéridine dans un mélange de 26 ml de dichlorométhane et de 5 ml d'acétonitrile est mise sous agitation pendant 24 heures. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le N-[5-({(2S)-3-[benzyl(4-{4-[(4-benzylpipéridin-1-yl)carbonyl]phényl}cyclohexyl)amino]-2-hydroxypropyl}oxy)-2-(benzyloxy)phényl] méthane sulfonamide est obtenu sous forme d'un solide blanc (1.355 g, 57%) après purification sur gel de silice (éluant : dichlorométhane/méthanol 90/10). [M+H⁺] = 816.6.

### 1.2 : Trans N-[5-({(2S)-3-[(4-{4-[(4-benzylpipéridin-1-yl)carbonyl]phényl} cyclohexyl)amino]-2-hydroxypropyl}oxy)-2-hydroxyphényl] méthane sulfonamide

Une suspension de 1.35 g (1.65 mmol) de N-[5-({(2S)-3-[benzyl(4-{4-[(4-benzylpipéridin-1-yl)carbonyl]phényl}cyclohexyl)amino]-2-hydroxypropyl}oxy)-2-(benzyloxy)phényl] méthane sulfonamide et de 0.77g de palladium sur charbon (10% Pd, 50% dans l'eau) dans 130ml d'éthanol est mise sous atmosphère d'hydrogène et sous agitation pendant deux heures. Le catalyseur est ensuite filtré et les solvants évaporés sous pression réduite. Le composé du titre est obtenu sous forme d'un solide blanc (0.53 g) après purification sur gel de silice (éluant : gradient, dichlorométhane / méthanol / ammoniaque, 99/1/0.1 à 85/15/1.5).
Rendement = 50% ; Point de fusion = 100-110°C; [M+H⁺] = 636.6 ; RMN ¹H (CDCl₃+ D₂O, 300 MHz) :1.1-2.05 (m, 14H), 2.02-2.18 (m, 3H), 2.45-2.7 (m, 5H), 2.7-2.9 (m, 2H), 2.95 (s, 3H), 3.7-3.95 (m, 3H), 4-4.1 (m, 1 H), 4.6-4.8 (bm, 1 H), 6.55 (dd,1 H), 7.0-7.3 (m, 9H).

### Exemple 2: Trans N-[2-hydroxy-5-({(2S)-2-hydroxy-3-[(4-{4-[(4-méthylpipéridin-1-yl)carbonyl]phényl}cyclohexyl)amino]propyl}oxy)phényl]méthane sulfonamide (composé n° 3)

En opérant comme décrit dans l'exemple 1, mais en utilisant la 4-méthyl-pipéridine dans l'étape 1.1, on obtient le composé du titre (42 mg) sous forme d'un solide blanc.
Rendement = 16% ; Point de fusion = 90-100°C ; [M+H⁺] = 560.4 ; RMN ¹H (DMSO-D6, 300M Hz) :0.8-2.05 (m, 14H), 0.88 (d, 3H), 2.3-3 (m, 5H), 3.1-3.5 (m, 3H), 2.90 (s, 3H), 3.7-3.95 (m, 3H), 6.55 (dd,1 H), 6.7-6.82 (m,3H), 7.18-7.32 (m, 4H).

### Exemple 3 : Trans 4-{[(2S)-2-hydroxy-3-({4-[4-({4-[hydroxy(phényl)méthyl] pipéridin-1-yl}carbonyl)phényl]cyclohexyl}amino)propyl]oxy}-2-(méthylsulfonyl) phénol (composé n° 9)

En opérant comme décrit dans l'exemple 1, mais en utilisant l'acide trans 4-[4-(benzyl{(2S)-3-[4-(benzyloxy)-3-(méthylsulfonyl)phénoxy]-2-hydroxypropyl}amino) cyclohexyl]benzoïque (Préparation 2) et la phényl(pipéridin-4-yl)méthanone dans l'étape 1.1, on obtient le composé du titre (20 mg) sous forme d'un solide blanc.
Rendement = 7.5% ; Point de fusion = 120°C ; [M+H⁺] = 637 ; RMN ¹H (DMSO-D6 + D₂O, 500 MHz) :1-1.35 (m, 5H), 1.45 (dd, 2H), 1.65-1.85 (m, 4H), 1.95-2.05 (m, 2H), 2.45-2.56 (m, 2H), 2.6-2.7 (m, 1 H), 2.75-2.8 (m,1 H), 2.82-2.95 (m, 1 H), 3.19 (s, 3H), 3.45-3.6 (m, 1 H), 3.8-3.9 (m, 3H), 4.28 (d, 1 H), 4.3-4.45 (m, 1 H), 6.88 (d,1 H), 7.1 (d, 1 H), 7.12-7.2 (m, 10H).

### Exemple 4 : Trans 4-{[(2S)-3-({4-[4-(1,3-dihydro-2H-isoindol-2-yl-carbonyl) phényl]cyclohexyl}amino)-2-hydroxypropyl]oxy}-2-(méthylsulfonyl)phénol (composé n° 11)

En opérant comme décrit dans l'exemple 1, mais en utilisant l'acide trans 4-[4-(benzyl{(2S)-3-[4-(benzyloxy)-3-(méthylsulfonyl)phénoxy]-2-hydroxypropyl}amino) cyclohexyl]benzoïque (Préparation 2) et l'isoindoline dans l'étape 1.1, on obtient le composé du titre (34 mg) sous forme d'un solide blanc.
Rendement = 14% ; Point de fusion = 110°C; [M+H⁺] = 565 ; RMN ¹H (DMSO-D6 + D₂O, 500 MHz) :1.23 (dd, 2H), 1.47 (dd, 2H), 1.82 (bd, 2H), 2 (bs, 2H), 2.49-2.58 (m, 1 H), 2.6-2.7 (m, 1 H), 2.75-2.8 (m,1 H), 3.19 (s, 3H), 3.8-3.95 (m, 3H), 4.76 (s, 2H), 4.83 (s, 2H), 6.9 (d,1 H), 7.22 (d, 1 H), 7.19 (s,1 H), 7.2-7.4 (m, 6H), 7.5 (d, 2H).

### Exemple 5: Trans 4-{[(2S)-3-({4-[4-(1,3-dihydro-1'H-spiro[indène-2,4'-pipéridin]-1'-yl-carbonyl)phényl]cyclohexyl}amino)-2-hydroxypropyl]oxy}-2-(méthylsulfonyl)phénol (composé n° 13)

En opérant comme décrit dans l'exemple 1, mais en utilisant l'acide trans 4-[4-(benzyl{(2S)-3-[4-(benzyloxy)-3-(méthylsulfonyl)phénoxy]-2-hydroxypropyl}amino) cyclohexyl]benzoïque (Préparation 2) et la 1,3-dihydrospiro[indène-2,4'-pipéridine] dans l'étape 1.1, on obtient le composé du titre (79 mg) sous forme d'un solide blanc.
Rendement = 28% ; Point de fusion = 125°C; [M+H⁺] = 633 ; RMN ¹H (DMSO-D6 + D₂O, 500 MHz) :1.17-1.3 (m, 2H), 1.35-1.65 (m, 4H), 1.68-1.85 (m, 4H), 1.9-2.12 (m, 4H), 2.48-2.6 (m, 1 H), 2.64-2.7 (m, 1 H), 2.76-2.8 (m,1 H), 2.81-2.9 (m, 2H), 2.91-3.03 (m, 1 H), 3.2 (s, 3H), 3.19-3.29 (m, 1 H), 3.5-3.65 (m, 1 H), 3.85-3.91 (m, 2H), 4.35-4.5 (m, 1 H), 6.92 (d,1 H), 7.1-7.22 (m, 6H), 7.25-7.38 (m, 4H).

### Exemple 6: Trans 4-{[(2S)-3-({4-[4-(2,3-dihydro-1'H-spiro[indène-1,4'-pipéridin]-1'-yl-carbonyl)phényl]cyclohexyl}amino)-2-hydroxypropyl]oxy}-2-(méthylsulfonyl)phénol (composé n° 15)

En opérant comme décrit dans l'exemple 1, mais en utilisant l'acide trans 4-[4-(benzyl{(2S)-3-[4-(benzyloxy)-3-(méthylsulfonyl)phénoxy]-2-hydroxypropyl}amino) cyclohexyl]benzoïque (Préparation 2) et la 1'H-spiro[indène-1,4'-pipéridine] dans l'étape 1.1, on obtient le composé du titre (68 mg) sous forme d'un solide blanc.
Rendement = 24% ; Point de fusion = 125°C; [M+H⁺] = 633 ; RMN ¹H (DMSO-D6 + D₂O, 500 MHz) :1.17-1.3 (m, 2H), 1.35-1.65 (m, 4H), 1.68-1.85 (m, 4H), 1.9-2.12 (m, 4H), 2.48-2.6 (m, 1H), 2.64-2.7 (m, 1H), 2.76-2.8 (m,1H), 2.81-2.9 (m, 2H), 2.91-3.03 (m, 1H), 3.2 (s, 3H), 3.19-3.29 (m, 1 H), 3.5-3.65 (m, 1 H), 3.79-3.85 (m, 1 H), 3.85-3.91 (m, 1 H), 4.37-4.5 (m, 1 H), 6.92 (d,1 H), 7.1-7.22 (m, 6H), 7.25-7.38 (m, 4H).

### Exemple 7 : Trans 4-{[(2S)-3-({4-[4-(3,4-dihydro-1'H,2H-spiro[naphthalène-1,4'-pipéridin]-1'-yl-carbonyl)phényl]cyclohexyl}amino)-2-hydroxypropyl]oxy}-2-(méthylsulfonyl)phénol (composé n° 17)

En opérant comme décrit dans l'exemple 1, mais en utilisant l'acide trans 4-[4-(benzyl{(2S)-3-[4-(benzyloxy)-3-(méthylsulfonyl)phénoxy]-2-hydroxypropyl}amino) cyclohexyl]benzoïque (Préparation 2) et la 3,4-dihydro-2H-spiro[naphthalène-1,4'-pipéridine] dans l'étape 1.1, on obtient le composé du titre (93 mg) sous forme d'un solide blanc.
Rendement = 35% ; Point de fusion = 125°C ; [M+H⁺] = 647 ; RMN ¹H (DMSO-D6 + D₂O, 500 MHz) :0.8-2.1 (m, 18H), 2.35-3 (m, 4H), 3.2 (s, 3H), 3.32 (bs, 1 H), 3.62-3.95 (m, 5H), 4.25-4.5 (m, 1 H), 6.9 (d,1 H), 6.9-7.52 (m, 10H).

Le tableau qui suit illustre les structures chimiques et les propriétés physiques de quelques composés selon l'invention. Dans ce tableau :
- dans la colonne « sel », « - » représente un composé sous forme de base libre,
- Me, Et, Ph et Bn représentent respectivement les groupes méthyle, éthyle, phényle et benzyle.

**Tableau**

| | | | | | |
|---|---|---|---|---|---|
| 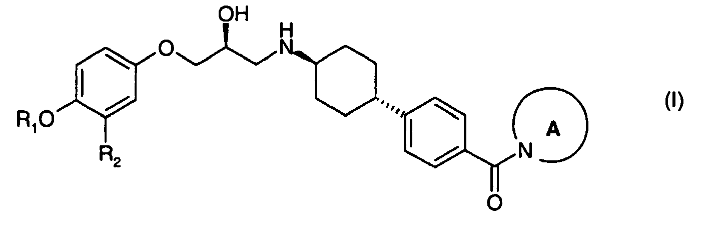 | | | | | |

| **N°** | **R₁** | **R₂** | **A** | **Sel** | **Point de fusion (°C)** |
|---|---|---|---|---|---|
| **1** | H | -NHSO₂CH₃ | 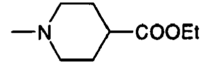 | pamoate | 110-160 |
| **2** | H | -NHSO₂CH₃ | 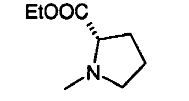 | pamoate | 110-150 |
| **3** | H | -NHSO₂CH₃ | 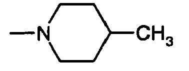 | - | 90-100 |
| **4** | H | -NHSO₂CH₃ | 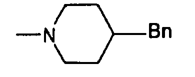 | - | 100-110 |
| **5** | H | -SO₂CH₃ | 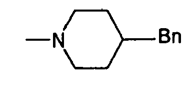 | - | 108-110 |
| **6** | H | -SO₂CH₃ | 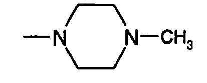 | - | 85 |
| **7** | H | -SO₂CH₃ | 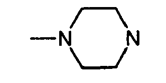 | - | 110 |
| **8** | H | -SO₂CH₃ | 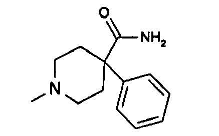 | - | 140 |
| **9** | H | -SO₂CH₃ | 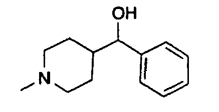 | - | 120 |
| **10** | H | -NHSO₂CH₃ | 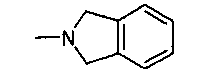 | - | 100 |
| **11** | H | -SO₂CH₃ | 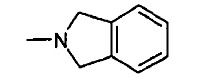 | - | 110 |
| **12** | H | -SO₂CH₃ | 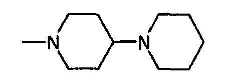 | - | 75 |
| **13** | H | -SO₂CH₃ | 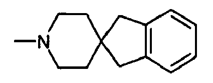 | - | 125 |
| **14** | H | -NHSO₂CH₃ | 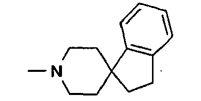 | - | 140 |
| **15** | H | -SO₂CH₃ | 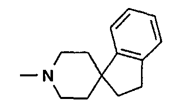 | - | 125 |
| **16** | H | -NHSO₂CH₃ | 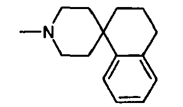 | - | 125 |
| **17** | H | -SO₂CH₃ | 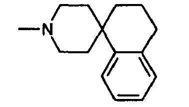 | - | 125 |
| **18** | H | -NHSO₂CH₃ | 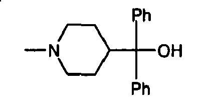 | - | 135 |
| **19** | H | -SO₂CH₃ | 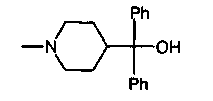 | - | 140 |
| **20** | H | -NHSO₂CH₃ | 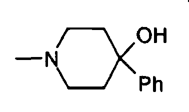 | - | 97 |
| **21** | H | -SO₂CH₃ | 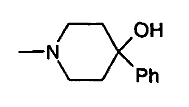 | - | 100 |
| **22** | H | -SO₂CH₃ | 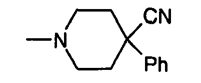 | - | 110 |
| **23** | H | -NHSO₂CH₃ | 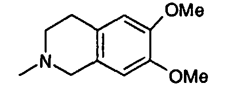 | - | 126 |
| **24** | H | -SO₂CH₃ | 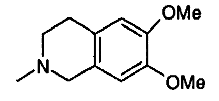 | - | 112 |
| **25** | H | -SO₂CH₃ | 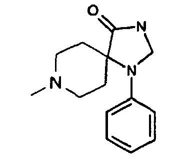 | - | 135 |

Les composés selon l'invention ont fait l'objet d'essais pharmacologiques permettant de déterminer leur effet activité agoniste vis-à-vis des récepteurs bêta-3.
L'activité agoniste vis-à-vis des récepteurs bêta-3 (traduite par la production de cAMP induite par le composé testé) a été étudiée à l'aide de préparations membranaires de cellules SKNMC (cellules de neuroblastomes humains) en présence d'antagonistes sélectifs bêta-1 et bêta-2 (CGP20712 et ICI118551, tous deux à une concentration de 10⁻⁶ M). L'activité des composés selon l'invention (pKa) est supérieure ou égale à 6.0 (elle est en général comprise entre 6.0 et 7.6). Leur efficacité est supérieure ou égale à 60% et se situe en général dans une gamme de 60 à 90%.
Les activités des composés selon l'invention envers les récepteurs bêta-1 et bêta-2 ont été étudiées sur l'oreillette et sur la trachée, respectivement, de cochons d'Inde. Les activités agonistes et antagonistes ont été mesurées. On a ainsi trouvé que les composés selon l'invention sont sélectifs envers les récepteurs bêta-3 : en effet, ils sont au moins 50 fois plus actifs envers les récepteurs bêta-3 qu'envers les récepteurs bêta-1 ou bêta-2.

Les composés selon l'invention peuvent donc être utilisés pour la préparation de médicaments, destinés notamment au traitement des maladies dans lesquelles les récepteurs bêta-3 sont impliqués. Plus particulièrement, les composés selon l'invention peuvent être utilisés comme médicaments à action bêta-3 agoniste.

Ainsi, selon un autre de ses aspects, l'invention a pour objet un médicament qui comprend un composé de formule (I), ou un sel d'addition de ce dernier à un acide pharmaceutiquement acceptable, ou encore un hydrate ou un solvat du composé de formule (I).

Des exemples de maladies dans lesquelles les récepteurs bêta-3 sont impliqués sont abondamment décrites dans littérature. Les composés de formule (I), ainsi que leurs sels pharmaceutiquement acceptables, ou hydrates ou solvats de ces composés, peuvent donc être indiqués pour le traitement des maladies gastro-intestinales, telles que les maladies inflammatoires de l'intestin, par exemple le syndrome du côlon irritable (IBS) ou la maladie du côlon inflammatoire (IBD), comme modulateurs de la motricité intestinale, comme lipolytiques, agents anti-obésité, anti-diabétiques, anti-glaucomateux, cicatrisants, comme inhibiteurs des contractions utérines, comme tocolytiques pour prévenir ou retarder les accouchements précoces, ainsi que pour le traitement et/ou la prophylaxie de la dysménorrhée. En outre, les composés de formule (I), ainsi que leurs sels pharmaceutiquement acceptables, ou hydrates ou solvats de ces composés, peuvent être utilisés dans le traitement de certaines maladies du système nerveux central, par exemple comme psychotropes ou anti-dépresseurs, ainsi que de certains troubles du système urinaire, tel que l'incontinence urinaire.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables, ou un hydrate ou solvat de ce composé.

La présente invention concerne également des compositions pharmaceutiques comprenant, en tant que principe actif, au moins un composé selon l'invention. Ces compositions pharmaceutiques contiennent une dose efficace d'un composé selon l'invention, ou un sel pharmaceutiquement acceptable, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

La dose de principe actif administrée par se situe entre 0,01 et 20 mg par kilo de poids corporel du mammifère à traiter, de préférence entre 0,1 et 10 mg/kg. Chez l'être humain, la dose peut varier de 0,5 mg à 1500 mg par jour, par exemple de 2,5 à 500 mg selon l'âge du sujet à traiter, le type de traitement (prophylactique ou curatif) et la gravité de l'affection. Les composés de formule (I) sont généralement administrés en forme unitaire de dosage de 0,1 à 500 mg, de préférence de 0,5 à 100 mg de principe actif, entre une et cinq fois par jour.
Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composés répondant à la formule (I): 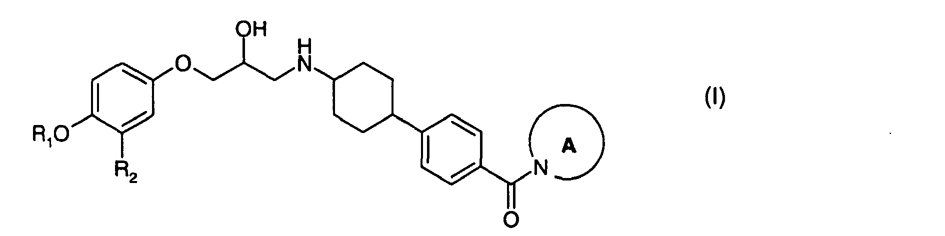 dans laquelle :
**R₁** représente un atome d'hydrogène ou un groupe (C1-C4)alkyle, un groupe -CO(C1-C4)alkyle, un groupe (C1-C4)alkyl-phényle ou un groupe -CO-phényle, ledit phényle étant éventuellement substitué par un à trois groupes choisis indépendamment les uns des autres parmi les atomes d'halogène, les groupes (C1-C4)alkyles et (C1-C4)alcoxy;
**R₂** est choisi parmi l'un des groupes suivants:
. un atome d'hydrogène,
. un atome d'halogène,
. un groupe -S(O)_{z}R₃,
. un groupe -NHSO₂R₃,
. un groupe -NHSO₂-phényle, ou
. un groupe -NHSO₂-(C1-C4)alkyl-phényle,
où z est égal à 0, 1 ou 2 et où R₃ représente un groupe (C1-C4)alkyle, ledit phényle étant éventuellement substitué par un à trois groupes choisis indépendamment les uns des autres parmi les atomes d'halogène, les groupes (C1-C4)alkyles et (C1-C4)alcoxy ; et
**A** est choisi parmi l'un des groupes de formule : 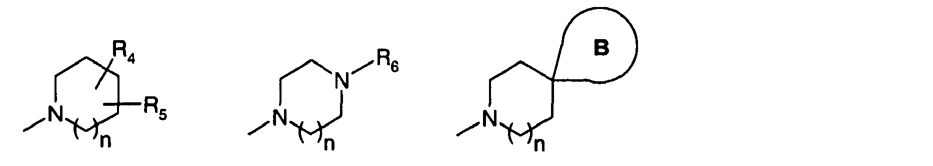 dans lesquelles:
. n est égal à 0, 1 ou 2,
. R₄ et R₅ sont portés soit par des atomes de carbone différents, soit par le même atome de carbone du cycle auquel ils sont rattachés, et sont choisis indépendamment l'un de l'autre parmi les groupes suivants : un atome d'hydrogène, un groupe (C1-C4)alkyle, hydroxy, cyano, phényle, benzyle, pipéridyle, -CONH₂, -CO-phényle, -COOR₃ (où R₃ est tel que défini ci-dessus), -CH(phényl)(OH) et -C(phényl)₂(OH), au moins l'un de R₄ ou R₅ étant différent d'un atome d'hydrogène,
. soit R₄ et R₅ sont portés par des atomes de carbone adjacents du cycle auquel ils sont rattachés et forment ensemble, avec les atomes de carbone qui les portent, un cycle aromatique à 6 chaînons éventuellement substitué par 1 à 3 groupes (C1-C4)alkyles ou (C1-C4)alcoxy,
. R₆ représente un atome d'hydrogène ou un groupe (C1-C4)alkyle, phényle ou benzyle, et
. B représente un groupe cycloalkyle à 5 ou 6 chaînons, saturé ou insaturé, comportant éventuellement 1 ou 2 atomes d'azote, ce groupe cycloalkyle étant lui-même fusionné avec un groupe phényle ou substitué par un à trois groupes choisis parmi les groupes phényle et carbonyle ;
à l'état de bases ou de sels d'addition à des acides, ainsi qu'à l'état d'hydrates ou de solvats.

2. Composés de formule (I) selon la revendication 1, **caractérisés en ce que** R₁ représente un atome d'hydrogène,
à l'état de bases ou de sels d'addition à des acides, ainsi qu'à l'état d'hydrates ou de solvats.

3. Composés de formule (I) selon la revendication 1 ou la revendication 2, **caractérisés en ce que** R₂ représente un groupe -SO₂R₃ ou -NHSO₂R₃, où R₃ est tel que défini dans la revendication 1,
à l'état de bases ou de sels d'addition à des acides, ainsi qu'à l'état d'hydrates ou de solvats.

4. Composés de formule (I) selon l'une quelconque des revendications 1 à 3, **caractérisés en ce que** A est choisi parmi l'un des groupes suivants:
. un groupe de formule 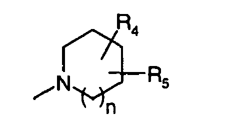
dans laquelle n est égal à 0 ou 1 et R₄ et R₅ sont portés soit par des atomes de carbone différents, soit par le même atome de carbone du cycle auquel ils sont rattachés, et sont choisis indépendamment l'un de l'autre parmi les groupes suivants : un atome d'hydrogène, un groupe (C1-C4)alkyle, hydroxy, cyano, phényle, benzyle, pipéridyle, -CONH₂, -CO-phényle, -COOR₃ (où R₃ est tel que défini dans la revendication 1), -CH(phényl)(OH) et -C(phényl)₂(OH), au moins l'un de R₄ ou R₅ étant différent d'un atome d'hydrogène,
. un groupe de formule 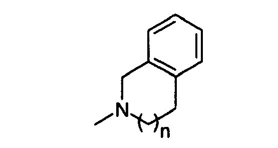
dans laquelle n est égal à 0 ou 1 et le cycle aromatique est éventuellement substitué par 1 à 3 groupes choisis indépendamment les uns des autres parmi les groupes (C1-C4)alkyles et (C1-C4)alcoxy,
. un groupe de formule 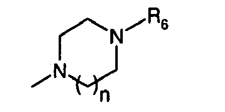
dans laquelle n est égal à 0 ou 1 et R₆ représente un atome d'hydrogène ou un groupe benzyle, et
. un groupe de formule 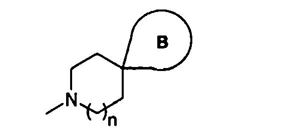
dans laquelle n est égal à 0 ou 1 et B représente un groupe cycloalkyle à 5 ou 6 chaînons, saturé ou insaturé, comportant éventuellement 1 ou 2 atomes d'azote, ce groupe cycloalkyle étant lui-même fusionné avec un groupe phényle ou substitué par un à trois groupes choisis parmi les groupes phényle et carbonyle ;
à l'état de bases ou de sels d'addition à des acides, ainsi qu'à l'état d'hydrates ou de solvats.

5. Procédé de préparation des composés de formule (I) selon l'une quelconque des revendications 1 à 4, **caractérisé en ce que** l'on fait réagir un composé de formule (VI), dans laquelle R₁ et R₂ sont tels que définis dans l'une quelconque des revendications 1 à 3 et Pg représente un groupe protecteur : 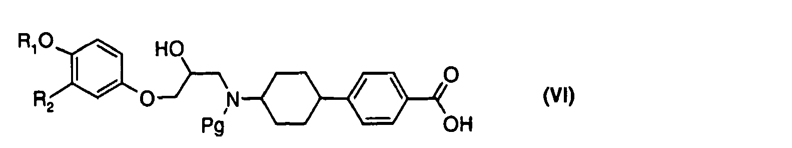 avec une amine de formule : 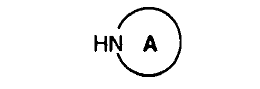 dans laquelle A est tel que défini dans les revendications 1 ou 4, en présence d'un agent de couplage et d'une base, puis que l'on élimine le groupe protecteur Pg du produit ainsi obtenu.

6. Médicament, **caractérisé en ce qu'**il comprend un composé de formule (I) selon l'une quelconque des revendications 1 à 4, ou un sel d'addition de ce composé à un acide pharmaceutiquement acceptable, ou encore un hydrate ou un solvat du composé de formule (I).

7. Composition pharmaceutique, **caractérisée en ce qu'**elle comprend un composé de formule (I) selon l'une quelconque des revendications 1 à 4, ou un sel pharmaceutiquement acceptable, un hydrate ou un solvat de ce composé, ainsi qu'au moins un excipient pharmaceutiquement acceptable.

8. Utilisation d'un composé de formule (I) selon l'une quelconque des revendications 1 à 4 pour la préparation d'un médicament destiné au traitement des maladies dans lesquelles les récepteurs bêta-3 sont impliqués.

9. Utilisation d'un composé de formule (I) selon l'une quelconque des revendications 1 à 4 pour la préparation d'un médicament destiné au traitement des maladies gastro-intestinales, telles que les maladies inflammatoires de l'intestin, par exemple le syndrome du côlon irritable (IBS) ou la maladie du côlon inflammatoire (IBD), comme modulateurs de la motricité intestinale, comme lipolytiques, agents anti-obésité, anti-diabétiques, anti-glaucomateux, cicatrisants, comme inhibiteurs des contractions utérines, comme tocolytiques pour prévenir ou retarder les accouchements précoces, pour le traitement et/ou la prophylaxie de la dysménorrhée, comme psychotropes ou anti-dépresseurs, ainsi que pour le traitement de l'incontinence urinaire.

10. Composé de formule (I) selon les revendications 1, 2, 3 ou 4 **caractérisé en ce qu'**il consiste en le :
• **Trans N-[5-({(2S)-3-[(4-{4-[(4-benzylpipéridin-1-yl)carbonyl] phényl}cyclohexyl)amino]-2-hydroxypropyl}oxy)-2-hydroxyphényl] méthane sulfonamide ;**
• **Trans N-[2-hydroxy-5-({(2S)-2-hydroxy-3-[(4-{4-[(4-méthyl-pipéridin-1-yl)carbonyl]phényl}cyclohexyl)amino]propyl}oxy)phényl] méthane sulfonamide ;**
• **Trans 4-{[(2S)-2-hydroxy-3-({4-[4-({4-[hydroxy(phényl)méthyl] pipéridin-1-yl}carbonyl)phényl]cyclohexyl}amino)propyl]oxy}-2-(méthylsulfonyl) phénol** ;
• **Trans 4-{[(2S)-3-({4-[4-(1,3-dihydro-2H-isoindol-2-yl-carbonyl) phényl]cyclohexyl}amino)-2-hydroxypropyl]oxy}-2-(méthylsulfonyl)phénol** ;
• **Trans 4-{[(2S)-3-({4-[4-(1,3-dihydro-1'H-spiro[indène-2,4'-pipéridin]-1'-yl-carbonyl)phényl]cyclohexyl}amino)-2-hydroxypropyl]oxy}-2-(méthylsulfonyl)phénol ;**
• **Trans 4-{[(2S)-3-({4-[4-(2,3-dihydro-1'H-spiro[indène-1,4'-pipéridin]-1'-yl-carbonyl)phényl]cyclohexyl}amino)-2-hydroxypropyl]oxy}-2-(méthylsulfonyl)phénol ou**
• **Trans 4-{[(2S)-3-({4-[4-(3,4-dihydro-1'H,2H-spiro[naphthalène-1,4'-pipéridin]-1'-yl-carbonyl)phényl]cyclohexyl}amino)-2-hydroxypropyl]oxy}-2-(méthylsulfonyl)phénol ;**
à l'état de base ou de sel d'addition, ainsi qu'à l'état d'hydrate ou de solvate.

## Claims

1. Compounds corresponding to formula (I): 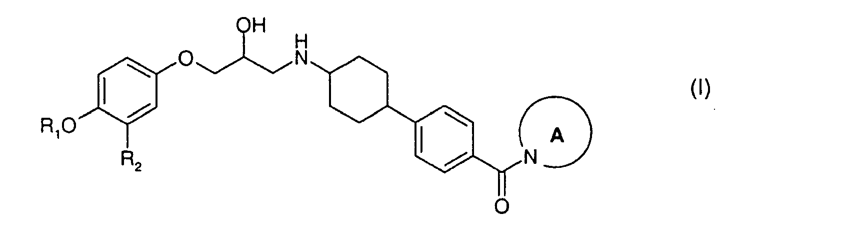 in which:
**R₁** represents a hydrogen atom or a (C1-C4)alkyl group, a -CO(C1-C4)alkyl group, a (C1-C4)alkylphenyl group or a -CO-phenyl group, said phenyl being optionally substituted with one to three groups chosen independently of each other from halogen atoms, (C1-C4)alkyl and (C1-C4)alkoxy groups;
**R₂** is chosen from one of the following groups:
. a hydrogen atom,
. a halogen atom,
. an -S(O)_{z}R₃ group,
. an -NHSO₂R₃ group,
. an -NHSO₂-phenyl group, or
. an -NHSO₂-(C1-C4)alkylphenyl group,
where z is equal to 0, 1 or 2 and where R₃ represents a (C1-C4)alkyl group, said phenyl being optionally substituted with one to three groups chosen independently of each other from halogen atoms, (C1-C4)alkyl and (C1-C4)alkoxy groups; and
**A** is chosen from one of the groups of formula: 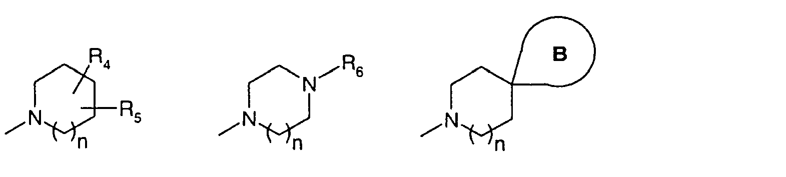 in which:
. n is equal to 0, 1 or 2,
. R₄ and R₅ are carried either by different carbon atoms, or by the same carbon atom of the ring to which they are attached, and are chosen independently of each other from the following groups: a hydrogen atom, a (C1-C4)alkyl, hydroxyl, cyano, phenyl, benzyl, piperidyl, -CONH₂, -CO-phenyl, -COOR₃ (where R₃ is as defined above), -CH(phenyl)(OH) and -C(phenyl)₂(OH) group, at least one of R₄ or R₅ being different from a hydrogen atom,
. or R₄ and R₅ are carried by adjacent carbon atoms of the ring to which they are attached and form together with the carbon atoms carrying them a 6-membered aromatic ring optionally substituted with 1 to 3 (C1-C4)alkyl or (C1-C4)alkoxy groups,
. R₆ represents a hydrogen atom or a (C1-C4)alkyl, phenyl or benzyl group, and
. B represents a saturated or unsaturated, 5- or 6-membered cycloalkyl group optionally containing 1 or 2 nitrogen atoms, this cycloalkyl group being itself condensed with a phenyl group or substituted with one to three groups chosen from phenyl and carbonyl groups;
in the form of bases or addition salts with acids, and in the form of hydrates or solvates.

2. Compounds of formula (I) according to Claim 1, **characterized in that** R₁ represents a hydrogen atom,
in the form of bases or addition salts with acids, and in the form of hydrates or solvates.

3. Compounds of formula (I) according to Claim 1 or Claim 2, **characterized in that** R₂ represents an -SO₂R₃ or -NHSO₂R₃ group, where R₃ is as defined in Claim 1,
in the form of bases or addition salts with acids, and in the form of hydrates or solvates.

4. Compounds of formula (I) according to any one of Claims 1 to 3, **characterized in that** A is chosen from one of the following groups:
. a group of formula 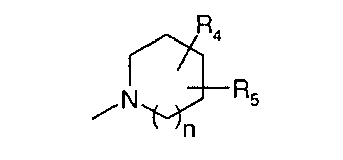
in which n is equal to 0 or 1 and R₄ and R₅ are carried either by different carbon atoms, or by the same carbon atom of the ring to which they are attached and are chosen independently of each other from the following groups: a hydrogen atom, a (C1-C4)alkyl, hydroxyl, cyano, phenyl, benzyl, piperidyl, -CONH₂, -CO-phenyl, -COOR₃ (where R₃ is as defined in Claim 1), -CH(phenyl)(OH) and -C(phenyl)₂(OH) group, at least one of R₄ or R₅ being different from a hydrogen atom,
. a group of formula 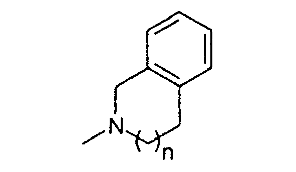
in which n is equal to 0 or 1 and the aromatic ring is optionally substituted with 1 to 3 groups chosen independently of each other from (C1-C4)alkyl and (C1-C4)alkoxy groups,
. a group of formula 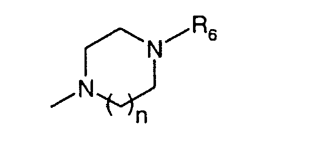
in which n is equal to 0 or 1 and R₆ represents a hydrogen atom or a benzyl group, and
. a group of formula 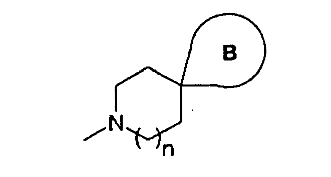
in which n is equal to 0 or 1 and B represents a saturated or unsaturated 5- or 6-membered cycloalkyl group optionally containing 1 or 2 nitrogen atoms, this cycloalkyl group being itself fused with a phenyl group or substituted with one to three groups chosen from phenyl and carbonyl groups;
in the form of bases or addition salts with acids, and in the form of hydrates or solvates.

5. Method for preparing the compounds of formula (I) according to any one of Claims 1 to 4, **characterized in that** a compound of formula (VI), in which R₁ and R₂ are as defined in any one of Claims 1 to 3 and Pg represents a protecting group: 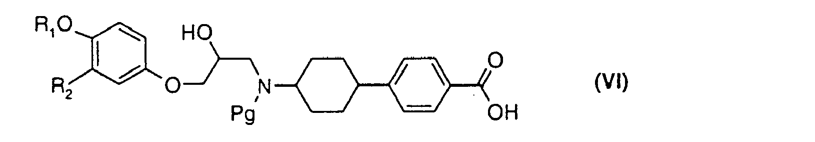 is reacted with an amine of formula: 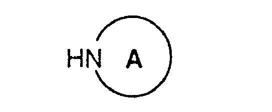 in which A is as defined in Claim 1 or 4, in the presence of a coupling agent and a base, and **in that** the protecting group Pg is removed from the product thus obtained.

6. Medicament, **characterized in that** it comprises a compound of formula (I) according to any one of Claims 1 to 4, or an addition salt of this compound with a pharmaceutically acceptable acid, or a hydrate or a solvate of the compound of formula (I).

7. Pharmaceutical composition, **characterized in that** it comprises a compound of formula (I) according to any one of Claims 1 to 4, or a pharmaceutically acceptable salt, a hydrate or a solvate of this compound, and at least one pharmaceutically acceptable excipient.

8. Use of a compound of formula (I) according to any one of Claims 1 to 4, for the preparation of a medicament intended for the treatment of diseases in which the beta-3 receptors are involved.

9. Use of a compound of formula (I) according to any one of Claims 1 to 4, for the preparation of a medicament intended for the treatment of gastrointestinal diseases such as inflammatory bowel diseases, for example irritable bowel syndrome (IBS) or inflammatory bowel disease (IBD), as modulators of intestinal motility, as lipolytics, anti-obesity, antidiabetic, anti-glaucomatous and cicatrizing agents, as uterine contraction inhibitors, as tocolytics for preventing or delaying preterm labour, and for the treatment and/or prophylaxis of dysmenorrhoea, as psychotropics or antidepressants, and for the treatment of urinary incontinence.

10. Compound of the formula (I) according to Claims 1, 2, 3 or 4, **characterized in that** it constitutes:
• **trans-N-[5-({(2S)-3-[(4-{4-[(4-benzylpiperidin-1-yl)carbonyl]phenyl}cyclohexyl)amino]-2-hydroxypropyl}oxy)-2-hydroxyphenyl]methanesulphonamide;**
• **trans-N-[2-hydroxy-5-({(2S)-2-hydroxy-3-[(4-{4-[(4-methylpiperidin-1-yl)carbonyl]phenyl}cyclohexyl)amino]propyl}oxy)phenyl]methanesulphonamide;**
• **trans-4-{[(2S)-2-hydroxy-3-({4-[4-({4-[hydroxy(phenyl)methyl]piperidin-1-yl}carbonyl)-phenyl]cyclohexyl}amino)propyl]oxy}-2-(methylsulphonyl)phenol;**
• **trans-4-{[(2S)-3-({4-[4-(1,3-dihydro-2H-isoindol-2-ylcarbonyl)phenyl]cyclohexyl}amino)-2-hydroxypropyl]oxy}-2-(methylsulphonyl)phenol;**
• **trans-4-{[(2S)-3-({4-[4-(1,3-dihydro-1'H-spiro[indene-2,4'-piperidin]-1'-ylcarbonyl)-phenyl]cyclohexyl}amino)-2-hydroxypropyl]oxy}-2-(methylsulphonyl)phenol;**
• **trans-4-{[(2S)-3-({4-[4-(2,3-dihydro-1'H-spiro[indene-1,4'-piperidin]-1'-ylcarbonyl)-phenyl]cyclohexyl}amino)-2-hydroxypropyl]oxy}-2-(methylsulphonyl)phenol or**
• **trans-4-{[(2S)-3-({4-[4-(3,4-dihydro-1'H,2H-spiro[naphthalene-1,4'-piperidin]-1'-ylcarbonyl)-phenyl]cyclohexyl}amino)-2-hydroxypropyl]oxy}-2-(methylsulphonyl)phenol;**
in the form of a base or addition salt, or in the form of a hydrate or solvate.

## Patentansprüche

1. Verbindungen der Formel (I) 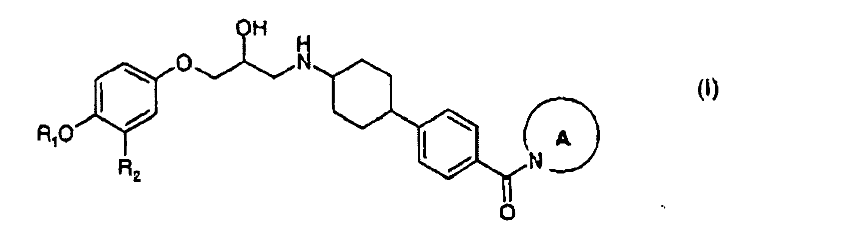 in der
R₁ ein Wasserstoffatom oder eine (C1-C4)Alkylgruppe, eine -CO(C1-C4)Alkylgruppe, eine (C1-C4)Alkylphenylgruppe oder eine -CO-Phenylgruppe bedeutet, wobei das Phenyl gegebenenfalls durch eine bis drei Gruppen, die unabhängig voneinander aus der Reihe Halogenatome, (C1-C4)Alkylgruppen und (C1-C4)Alkoxygruppen stammen, substituiert ist;
R₂ aus den folgenden Gruppen stammt:
Wasserstoffatom,
Halogenatom,
- S(O)₂R₃-Gruppe,
- NHSO₂R₃-Gruppe,
- NHSO₂-Phenyl-Gruppe oder
- NHSO₂-(C1-C4)Alkylphenyl-Gruppe,
wobei z gleich 0, 1 oder 2 ist und wobei R₃ eine (C1-C4)Alkylgruppe bedeutet, wobei das Phenyl gegebenenfalls durch eine bis drei Gruppen, die unabhängig voneinander aus der Reihe Halogenatome, (C1-C4)Alkylgruppen und (C1-C4)Alkoxygruppen stammen, substituiert ist, und
A aus der Reihe der Gruppen der Formel 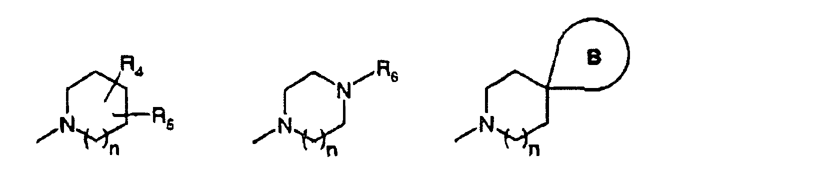 stammt,
in denen
n gleich 0, 1 oder 2 ist,
R₄ und R₅ entweder von verschiedenen Kohlenstoffatomen oder vom selben Kohlenstoffatom des Rings, an den sie gebunden sind, getragen werden und unabhängig voneinander aus der Reihe der folgenden Gruppen stammen: Wasserstoffatom, (C1-C4)Alkyl-, Hydroxy-, Cyano-, Phenyl-, Benzyl-, Piperidyl-, -CONH₂-, -CO-Phenyl-, -COOR₃- (wobei R₃ wie oben definiert ist), -CH(Phenyl)(OH)- und -C(Phenyl)₂(OH)-Gruppe, wobei sich mindestens einer der Reste R₄ oder R₅ vom Wasserstoffatom unterscheidet,
oder R₄ und R₅ von benachbarten Kohlenstoffatomen des Rings, an den sie gebunden sind, getragen werden und zusammen mit den Kohlenstoffatomen, die sie tragen, einen aromatischen Ring mit 6 Ringgliedern bilden, der gegebenenfalls durch 1 bis 3 (C1-C4)Alkylgruppen oder (C1-C4)Alkoxygruppen substituiert ist,
R₆ ein Wasserstoffatom oder eine (C1-C4)Alkyl-, Phenyl- oder Benzylgruppe bedeutet, und
B eine gesättigte oder ungesättigte Cycloalkylgruppe mit 5 oder 6 Ringgliedern bildet, die gegebenenfalls 1 oder 2 Stickstoffatome enthält, wobei diese Cycloalkylgruppe selbst wiederum mit einer Phenylgruppe anelliert ist oder durch eine bis drei Gruppen aus der Reihe der Phenyl- und Carbonylgruppen substituiert ist,
als Basen oder Additionssalze mit Säuren sowie als Hydrate oder Solvate.

2. Verbindungen der Formel (I) nach Anspruch 1, **dadurch gekennzeichnet, daß** R₁ ein Wasserstoffatom bedeutet,
als Basen oder Additionssalze mit Säuren sowie als Hydrate oder Solvate.

3. Verbindungen der Formel (I) nach Anspruch 1 oder Anspruch 2, **dadurch gekennzeichnet, daß** R₂ eine -SO₂R₃-Gruppe oder -NHSO₂R₃-Gruppe bedeutet, wobei R₃ wie in Anspruch 1 definiert ist,
als Basen oder Additionssalze mit Säuren sowie als Hydrate oder Solvate.

4. Verbindungen der Formel (I) nach einem der Ansprüche 1 bis 3, **dadurch gekennzeichnet, daß** A aus einer der folgenden Gruppen stammt:
Gruppe der Formel 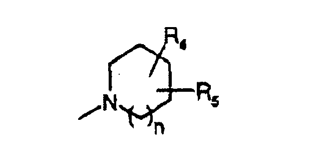
in der n gleich 0 oder 1 ist und R₄ und R₅ entweder von verschiedenen Kohlenstoffatomen oder vom selben Kohlenstoffatom des Rings, an den sie gebunden sind, getragen werden und unabhängig voneinander aus der Reihe der folgenden Gruppen stammen: Wasserstoffatom, (C1-C4)Alkyl-, Hydroxy-, Cyano-, Phenyl-, Benzyl-, Piperidyl-, -CONH₂-, -CO-Phenyl-, -COOR₃- (wobei R₃ wie in Anspruch 1 definiert ist), -CH(Phenyl)(OH)- und -C(Phenyl)₂(OH)-Gruppe, wobei sich mindestens einer der Reste R₄ oder R₅ vom Wasserstoffatom unterscheidet, Gruppe der Formel 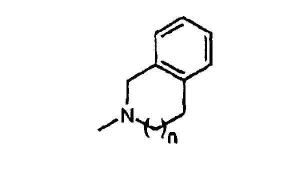 in der n gleich 0 oder 1 ist und der aromatische Ring gegebenenfalls durch 1 bis 3 Gruppen substituiert ist, die unabhängig voneinander aus der Reihe der (C1-C4)Alkyl- und (C1-C4)Alkoxygruppen stammen,
Gruppe der Formel 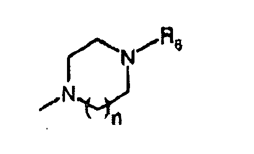 in der n gleich 0 oder 1 ist und R₆ ein Wasserstoffatom oder eine Benzylgruppe bedeutet, und
Gruppe der Formel 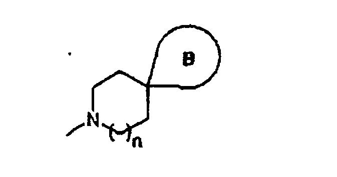 in der n gleich 0 oder 1 ist und B eine gesättigte oder ungesättigte Cycloalkylgruppe mit 5 oder 6 Ringgliedern, die gegebenenfalls 1 oder 2 Stickstoffatome enthält, wobei diese Cycloalkylgruppe selbst wiederum mit einer Phenylgruppe anelliert ist oder durch eine bis drei Gruppen aus der Reihe der Phenyl- und Carbonylgruppen substituiert ist;
als Basen oder Additionssalze mit Säuren sowie als Hydrate oder Solvate.

5. Verfahren zur Herstellung von Verbindungen der Formel (I) nach einem der Ansprüche 1 bis 4, **dadurch gekennzeichnet, daß** man eine Verbindung der Formel (VI), in der R₁ und R₂ nach einem der Ansprüche 1 bis 3 definiert sind und Sg eine Schutzgruppe bedeutet: 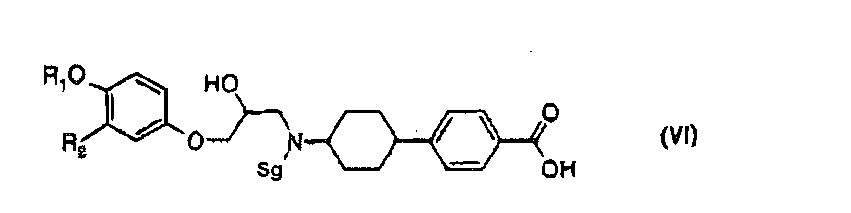 mit einem Amin der Formel 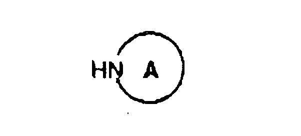 in der A gemäß den Ansprüchen 1 oder 4 definiert ist,
in Gegenwart eines Kopplungsmittels und einer Base umsetzt und anschließend die Schutzgruppe Sg vom so erhaltenen Reaktionsprodukt entfernt.

6. Arzneimittel, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure oder ein Hydrat oder Solvat der Verbindung der Formel (I) enthält.

7. Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz, ein Hydrat oder Solvat der Verbindung sowie mindestens einen pharmazeutisch unbedenklichen Grundstoff enthält.

8. Verwendung einer Verbindung der Formel (I) nach einem der Ansprüche 1 bis 4 zur Herstellung eines Arzneimittels zur Behandlung von Krankheiten, an denen die beta-3-Rezeptoren beteiligt sind.

9. Verwendung einer Verbindung der Formel (I) nach einem der Ansprüche 1 bis 4 zur Herstellung eines Arzneimittels zur Behandlung von Magen-Darm-Krankheiten wie entzündlichen Darmerkrankungen, zum Beispiel Reizdarm (IBS) oder chronisch-entzündliche Darmerkrankung (IBD), als Modulatoren der Darmbeweglichkeit, als Lipolytika, als Schlankmacher, als Antidiabetika, Antiglaukomamittel, Wundheilungsmittel, als Uteruskontraktionshemmer, als Wehenhemmer zur Vorbeugung gegen bzw. Hinauszögerung von vorzeitiger Niederkunft, zur Behandlung und/oder Prophylaxe von Dysmenorrhö, als Psychotropika oder Antidepressiva sowie für die Behandlung von Harninkontinenz.

10. Verbindungen der Formel (I) nach einem der Ansprüche 1, 2, 3 oder 4, **dadurch gekennzeichnet, daß** sie aus:
• trans-N-[5-({(2S)-3-[(4-{4-[(4-Benzylpiperidin-1-yl)carbonyl]phenyl}cyclohexyl)amino]-2-hydroxypropyl}oxy)-2-hydroxyphenyl]methansulfonamid;
• trans-N-[2-Hydroxy-5-({(2S)-2-hydroxy-3-[(4-{4-[(4-methylpiperidin-1-yl)carbonyl]phenyl}cyclohexyl)amino]propyl}oxy)phenyl]methansulfonamid;
• trans-4-{[(2S)-2-Hydroxy-3-({4-[4-({4-[hydroxy(phenyl)methyl]piperidin-1-yl}carbonyl)phenyl]cyclohexyl}amino)propyl]oxy}-2-(methylsulfonyl)phenol;
• trans-4-{[(2S)-3-({4-[4-(1,3-Dihydro-2H-isoindol-2-yl-carbonyl)phenyl]cyclohexyl}amino)-2-hydroxylpropyl]oxy}-2-(methylsulfonyl)phenol;
• trans-4-{[(2S)-3-({4-[4-(1,3-Dihydro-1'H-spiro[inden-2,4'piperidin]]-1'-yl-carbonyl)phenyl]cyclohexyl}amino)-2-hydroxypropyl]oxy}-2-(methylsulfonyl)phenol;
• trans-4-{[(2S)-3-({4-[4-(2,3-Dyhydro-1'H-spiro[inden-1,4'-piperidin]-1'-yl-carbonyl)phenyl]cyclohexyl}amino)-2-hydroxypropyl]oxy}-2-(methylsulfonyl)phenol
oder
• trans-4-{[(2S)-3-({4-[-(3,4-Dihydro-1'H,2H-spiro[naphtalen-1,4'-piperidin]-1'yl-carbonyl)phenyl]cyclohexyl}amino)-2-hydroxypropyl]oxy}-2-(methylsulfonyl)phenol;
als Basen oder Additionssalze sowie als Hydrate oder Solvate bestehen.
